# EUROPEAN PATENT SPECIFICATION

(11) **EP 2 289 502 B1**
(45) Date of publication and mention of the grant of the patent: **22.07.2015**
(21) Application number: 10011299.4
(22) Date of filing: 30.04.2004
(51) Int. Cl.: A61K 31/167, A61K 45/06, A61P 3/06, A61K 31/225, A61K 31/325, A61K 31/366, A61K 31/40, A61K 31/401

(54) **Combination comprising s-[2-([[1-(2-ethylbutyl)cyclohexyl]carbonyl]amino)phenyl]2-methylpropanethioate and an hmg coa reductase inhibitor**
Arzneimittel enthaltend s-[2-([[1-(2-ethylbutyl) cyclohexyl] carbonyl] phenyl] 2-methylpropionsäure-s und einen hmg-coa Reduktase hemmern
Combination comprenant s-[2-([[1-(2-ethylbutyl) cyclohexyl] carbonyl] phenyl] 2-methylpropanethioate et un inhibiteur de la hmg coa réductase

(30) Priority: 02.05.2003 US 467418 P; 16.05.2003 US 471495 P; 10.06.2003 US 477372 P; 08.01.2004 US 534856 P
(43) Date of publication of application: 02.03.2011
(62) Divisional of application: 04751157.1
(73) Proprietor: Japan Tobacco Inc., Tokyo 105-8422 (JP)
(72) Inventor: Urata, Yasuo, Tokyo 105-8422 (JP); Hoshino, Shoji, Tokyo 105-8422 (JP); Kawamura, Hitoshi, Yokohama Kanagawa 227-0051 (JP); Okamoto, Hiroshi, Takatsuki-shi, Osaka 569-1125 (JP); Furukawa, Noboru, Takatsuki-shi Osaka 569-1125 (JP)
(74) Representative: Vossius & Partner Patentanwälte Rechtsanwälte mbB

(56) References cited:
- WO-A-00/38722
- INAZU A ET AL: "Therapeutic implications of cholesteryl ester transfer protein inhibitors in hyperlipidemia and low high-density lipoprotein-cholesterolemia", CURRENT OPINION IN INVESTIGATIONAL DRUGS 01 MAR 2003 UNITED KINGDOM, vol. 4, no. 3, 1 March 2003 (2003-03-01), pages 291-297, XP009036381, ISSN: 1472-4472
- WIERZBICKI A S: "New lipid-lowering agents", EXPERT OPINION ON EMERGING DRUGS 2003 UNITED KINGDOM, vol. 8, no. 2, 2003, pages 365-376, XP009036380, ISSN: 1472-8214
- OKAMOTO HIROSHI ET AL: "Effect of JTT-705 on cholesteryl ester transfer protein and plasma lipid levels in normolipidemic animals.", EUROPEAN JOURNAL OF PHARMACOLOGY. 11 APR 2003, vol. 466, no. 1-2, 11 April 2003 (2003-04-11), pages 147-154, XP002288185, ISSN: 0014-2999

## Description

### FIELD OF THE INVENTION

This invention is directed to combinations, pharmaceutical compositions, and their use methods for the treatment or prophylaxis of cardiovascular disorders.

### BACKGROUND OF THE INVENTION

Hyperlipidemic conditions associated with elevated concentrations of total cholesterol and low-density lipoprotein (LDL) cholesterol are major risk factors for coronary heart disease, and atherosclerosis in particular. Additionally, numerous studies have demonstrated that a low plasma concentration of high-density lipoprotein (HDL) cholesterol is a powerful risk factor for the development of atherosclerosis.

Hydroxy-methylglutaryl coenzyme A reductase (i.e., HMG CoA reductase) is an enzyme in the liver that functions in the production of cholesterol. Inhibition of HMG CoA reductase by HMG CoA reductase inhibitors (i.e., "statins") has been shown to reduce the level of cholesterol in the blood by reducing the production and accelerating the uptake of cholesterol.

Cholesteryl ester transfer protein (CETP) is a plasma protein that facilitates the movement of cholesteryl esters and triglycerides between various lipoproteins in the blood. The movement of cholesteryl ester from HDL to Apo B-containing lipoprotein particles (including VLDL, IDL, and LDL) by CETP has the effect of lowering HDL cholesterol and increasing LDL cholesterol. Inhibition of CETP activity has been shown to effectively modify plasmid HDL/LDL ratios by elevating plasma HDL cholesterol and lowering plasma LDL cholesterol.

*S*-[2-([[1-(2-ethylbutyl)cyclohexyl]carbonyl]amino)phenyl] 2-methylpropanethioate has been shown to be an inhibitor of CETP activity in humans (de Grooth et al., Circulation, 105, 2159-2165 (2002)) and rabbits (Shinkai et al., J. Med. Chem., 43, 3566-3572 (2000); Kobayashi et al., Atherosclerosis, 162, 131-135 (2002); and Okamoto et al., Nature, 406(13), 203-207 (2000)). *S*-[2-([[1-(2-ethylbutyl)cyclohexyl]carbonyl]amino)phenyl] 2-methylpropanethioate has been shown to increase plasma HDL cholesterol in humans (de Grooth et al., *supra*) and in rabbits (Shinkai et al., *supra*; Kobayashi et al., *supra*; Okamoto et al., *supra*). Moreover, *S*-[2-([[1-(2-ethylbutyl)cyclohexyl]carbonyl]amino)phenyl] 2-methylpropanethioate has been shown to decrease LDL cholesterol in humans (de Grooth et al., *supra*) and rabbits (Okamoto et al., *supra). S*-[2-([[1-(2-ethylbutyl)cyclohexyl]carbonyl]amino)phenyl] 2-methylpropanethioate, as well as methods of making and using the compound, are described in U.S. Patent 6,426,365.

Similarly, HMG CoA reductase inhibitors, methods of making and using the compounds, pharmaceutical compositions comprising the compounds, and the use of the compounds to treat cardiovascular disorders have been described in, for example, U.S. Patents 4,346,277, 4,444,784, 4,681,893, 5,011,930, 5,030,447, 5,180,589, 5,260,440, 5,273,995, 5,354,772, 5,356,896, 5,622,985, 5,686,104, 5,916,595, 5,969,156, 6,080,778, 6,126,971, 6,242,003, RE36481, and RE36520.

Despite the existence and use of such compounds for the treatment or prophylaxis of cardiovascular disorders, there remains a need for improved compositions and methods for the treatment and prophylaxis of cardiovascular disorders. The present invention provides compositions and methods for treating cardiovascular disorders.

### BRIEF SUMMARY OF THE INVENTION

The invention provides a combination comprising (a) *S*-[2-([[1-(2-ethylbutyl)cyclohexyl]carbonyl]amino)phenyl] 2-methylpropanethioate, and (b) at least one HMG CoA reductase inhibitor.

The invention also provides a pharmaceutical composition comprising (a) *S*-[2-([[1-(2-ethylbutyl)cyclohexyl]carbonyl]amino)phenyl] 2-methylpropanethioate (b) at least one HMG CoA reductase inhibitor, and (c) one or more pharmaceutically acceptable carriers.

The invention further provides a package comprising separate dosage units, of which (a) at least one dosage unit comprises *S*-[2-([[1-(2-ethylbutyl)cyclohexyl]carbonyl]amino)phenyl] 2-methylpropanethioate and (b) at least one other dosage unit comprises an HMG CoA reductase inhibitor.

The invention additionally provides a kit comprising (a) a first pharmaceutical composition comprising a therapeutically effective amount of(i) *S*-[2-([[1-(2-ethylbutyl)cyclohexyl]carbonyl]amino)phenyl] 2-methylpropanethioate, and (ii) a pharmaceutically acceptable carrier, (b) a second pharmaceutical composition comprising (i) at least one HMG CoA reductase inhibitor, and (ii) a pharmaceutically acceptable carrier, (c) prescribing information, and (d) a container, wherein the first and second pharmaceutical compositions can be the same or different, and wherein the prescribing information includes advice to a patient regarding co-administration of *S*-[2-([[1-(2-ethylbutyl)cyclohexyl]carbonyl]amino)phenyl] 2-methylpropanethioate, and the HMG CoA reductase inhibitor.

The invention additionally discloses a method for the treatment or prophylaxis of a cardiovascular disorder in a patient, which comprises treating the patient with a therapeutically effective amount of a combination of (a) *S*-[2-([[1-(2-ethylbutyl)cyclohexyl]carbonyl]amino)phenyl]2-methylpropanethioate, and (b) at least one HMG CoA reductase inhibitor.

### DETAILED DESCRIPTION OF THE INVENTION

The invention is directed to a combination, pharmaceutical composition, package, kit, and use in a method for the treatment or prophylaxis of cardiovascular disorders. The combination, pharmaceutical composition, package, kit, and use comprise and/or involve the use of (a) *S*-[2-([[1-(2-ethylbutyl)cyclohexyl]carbonyl]amino)phenyl] 2-methylpropanethioate, and (b) at least one HMG CoA reductase inhibitor. The combination of the two compounds provides an enhanced effect relative to treatment with either of the compounds alone.

The cardiovascular disorders include, but are not limited to, cardiovascular disease, coronary heart disease, coronary artery disease, hypoalphalipoproteinemia (low levels of HDL cholesterol), hyperbetalipoproteinemia (high levels of LDL cholesterol), hypercholesterolemia, hyperlipidemia, and atherosclerosis. Additional cardiovascular disorders which can be treated or prevented include, but are not limited to, hypertension, hypertriglyceridemia, hyperlipidoproteinemia, peripheral vascular disease, angina, ischemia, primary hypercholesterolemia (homozygous and heterozygous familial and nonfamilial), mixed dylipidemis (Frederickson Types IIa and IIb), and myocardial infarction. Following treatment with the above-described combination, the progression of atherosclerotic plaques is preferably slowed or arrested (e.g., in coronary arteries, in carotid arteries, and/or in the peripheral arterial system) in a patient. Preferably, the atherosclerotic plaques regress following treatment (e.g., in coronary arteries, in carotid arteries, and/or in the peripheral arterial system) in a patient.

As used herein, the term "patient" refers to a human patient.

*S*-[2-([[1-(2-ethylbutyl)cyclohexyl]carbonyl]amino)phenyl] 2-methylpropanethioate (herein referred to as Compound I) has the following structural formula:

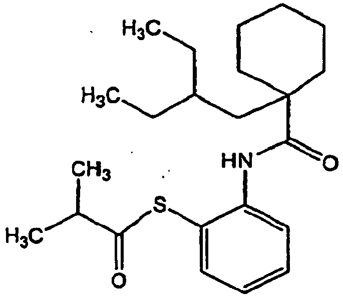

While not wishing to be bound by any particular theory, it is hypothesized that within the body of a patient, Compound I is hydrolyzed in plasma, the liver, and/or the small intestine to form *S*-[2([[1-(2-ethylbutyl)cyclohexyl]carbonyl]amino)phenyl] thiol (herein referred to as Compound II). It is known that low molecular weight thiol components (i.e., R-SH), such as cysteine and glutathione, and high molecular weight thiol components (i.e., Prot-SH), such as peptides and proteins (e.g., enzymes and cell membranes), exist in the body as mixed disulfides containing an oxidized disulfide bond (S-S bond) between or within the molecule (see, e.g., Shimada et al., J. Chromatogr. B, 659, 227 (1994)). Therefore, it is hypothesized that within the body of a patient (i.e., *in vivo*), Compound II is conjugated with low or high molecular weight thiols to yield mixed disulfides or to yield dimers of Compound II. Since these forms are in an oxidation-reduction equilibrium with each other via Compound II, all of these forms, as well as Compound II, are collectively, but not exclusively, considered and referred to hereafter as the active form of Compound I. The following scheme depicts the above-described hypothesis.

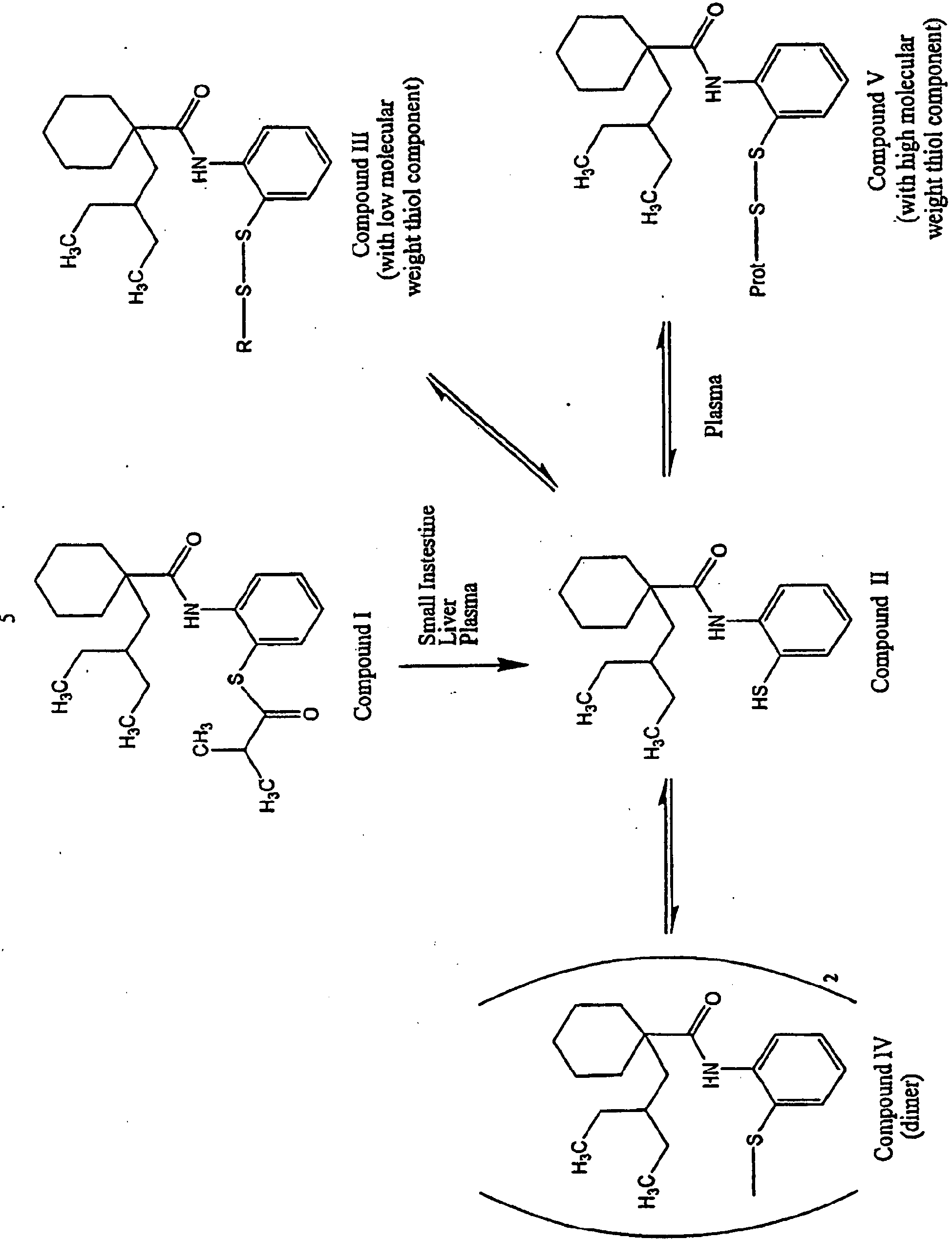

While the existence and/or administration of Compound I in combination with at least one HMG CoA reductase inhibitor is a particularly preferred embodiment of the invention. Also described the administration of other compounds that will yield the active form of Compound I, i.e., other prodrugs of the active form of Compound I, in combination with the HMG CoA reductase inhibitor. Such prodrugs, for example, can be compounds that have different mercapto-protecting groups, but that still result in the formation of the active form of Compound I (e.g., Compound II) in the body of a patient (i.e., *in vivo*). The term "mercapto-protecting groups" refers to commonly used mercapto-protecting groups (e.g., as described in Wolman, The Chemistry of the Thiol Group, D. Patai, Ed., Wiley-Interscience, New York, 1974). Any organic residues that can be dissociated *in vivo* may be used without particular restriction. Examples of particularly suitable mercapto-protecting groups are described in U.S. Patent 6,426,365. The invention further contemplates the administration of Compound r (wherein R' signifies an organic residue other than an isopropyl group) so as to yield the active form of Compound I, in combination with the HMG CoA reductase inhibitor.

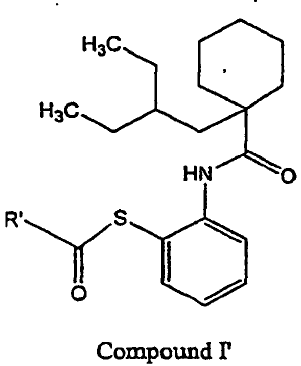

In addition, Compounds III, IV, and V (wherein R signifies an organic residue and Prot signifies a peptide or protein), which are believed to be in equilibrium with Compound II *in vivo,* similarly can be directly administered in combination with the HMG CoA reductase inhibitor to the patient.

Any suitable HMG CoA reductase inhibitor, which can be in the form of a pharmaceutically acceptable salt and/or solvate (e.g., hydrate), can be combined with Compound I or a prodrug of the active form of Compound I (e.g., a prodrug of Compound II). The HMG CoA reductase inhibitor can be in the form of a salt (e.g., atorvastatin calcium), which in turn can be in anhydrous form or a solvate, such as a hydrate (e.g., atorvastatin calcium (trihydrate)), or the HMG CoA reductase inhibitor can be in the form of a solvate (e.g., a hydrate), which may or may not be in the form of a salt. Suitable HMG CoA reductase inhibitors include, but are not limited to, atorvastatin (e.g., atorvastatin calcium (trihydrate); Lipitor^{™} available from Parke-Davis; Sortis^{™} available from Parke-Davis GmbH); pravastatin (e.g., pravastatin sodium; Pravachol^{™} available from Bristol-Myers Squibb; Selektine^{™} available from Bristol-Myers Squibb B.V.), fluvastatin (e.g., fluvastatin sodium; Lescol^{™} and Lescol XL^{™} available from Novartis), simvastatin (Zocor^{™} available from Merck), lovastatin (Mevacor^{™} available from Merck; Altocor^{™} available from Andrx Laboratories, Inc.), rosuvastatin (e.g., rosuvastatin calcium; Crestor^{™} available from AstraZeneca), and pitavastatin (e.g., pitavastatin calcium). Preferred HMG CoA reductase inhibitors include atorvastatin calcium (e.g., as atorvastatin calcium (trihydrate)), pravastatin sodium, fluvastatin sodium, simvastatin, lovastatin, rosuvastatin calcium, and pitavastatin calcium.

The combination of the invention comprises (a) Compound I, and (b) at least one HMG CoA reductase inhibitor. The combination of the invention can constitute one dosage unit comprising (a) Compound I and (b) at least one HMG CoA reductase inhibitor. Alternatively, the combination of the invention can constitute separate dosage units (such as different pharmaceutical compositions), wherein at least one dosage unit comprises Compound I, and at least one other dosage unit comprises at least one HMG CoA reductase inhibitor.

The pharmaceutical composition of the invention comprises the combination of the invention and one or more pharmaceutically acceptable carriers. In other words, the pharmaceutical composition comprises (a) Compound I, (b) at least one HMG CoA reductase inhibitor, and (c) one or more pharmaceutically acceptable carriers. The package of the invention contains separate dosage units, of which (a) at least one dosage unit comprises Compound I and (b) at least one other dosage unit comprises an HMG CoA reductase inhibitor. Each of the separate dosage units can comprise the aforementioned active ingredient of that separate dosage unit with one or more pharmaceutically acceptable carriers. A "package" is understood to be any package useful for stable storage of the dosage units. The package may, for example, be a glass or plastic (e.g., a high-density polyethylene) container generally used for packaging and storage of tablets. Another form of packaging is a blister pack. Blister packs are well known in the packaging industry and are being widely used for the packaging of pharmaceutical unit dosage forms (e.g., tablets, capsules, and the like). Blister packs generally consist of a sheet of relatively stiff material covered with a foil of a preferably transparent plastic material. During the packaging process recesses are formed in the plastic foil. The recesses have the size and shape of the tablets or capsules to be packed. Next, the tablets or capsules are placed in the recesses and the sheet of relatively stiff material is sealed against the plastic foil at the face of the foil which is opposite from the direction in which the recesses were formed. As a result, the tablets or capsules are sealed in the recesses between the plastic foil and the sheet. Preferably the strength of the sheet is such that the tablets or capsules can be removed from the blister pack by manually applying pressure on the recesses whereby an opening is formed in the sheet at the place of the recess. The tablet or capsule can then be removed via the opening.

The kit of the invention comprises (a) a first pharmaceutical composition comprising a therapeutically effective amount of (i) Compound I, and (ii) a pharmaceutically acceptable carrier, (b) a second pharmaceutical composition comprising (i) at least one HMG CoA reductase inhibitor, and (ii) a pharmaceutically acceptable carrier, (c) prescribing information, and (d) a container. The first and second pharmaceutical compositions can be the same (i.e., a single pharmaceutical composition) or different (i.e., two separate pharmaceutical compositions). The prescribing information includes advice to a patient regarding co-administration of (a) Compound I, and (b) the HMG CoA reductase inhibitor. The prescribing information, therefore, desirably includes advice to a patient regarding administration of Compound I. The prescribing information also desirably includes advice to a patient regarding administration of the HMG CoA reductase inhibitor. The prescribing information desirably advises the patient to administer within a 24-hour period (e.g., within an 18-hour period, or within a 12-hour period) both (a) Compound I, and (b) the HMG CoA reductase inhibitor.

Such a kit, therefore, has two primary embodiments. The first embodiment is a kit comprising (a) a pharmaceutical composition comprising a therapeutically effective amount of (i) Compound I, (ii) at least one HMG CoA reductase inhibitor, and (iii) one or more pharmaceutically acceptable carriers, (b) prescribing information, and (c) a container, wherein the prescribing information includes advice to a patient regarding co-administration of Compound I, and the HMG CoA reductase inhibitor. The second embodiment is a kit comprising (a) a first pharmaceutical composition comprising a therapeutically effective amount of (i) Compound I, and (ii) a pharmaceutically acceptable carrier, (b) a second pharmaceutical composition comprising (i) at least one HMG CoA reductase inhibitor, and (ii) a pharmaceutically acceptable carrier, (c) prescribing information, and (d) a container, wherein the first and second pharmaceutical compositions are different, and wherein the prescribing information includes advice to a patient regarding co-administration of Compound I, and the HMG CoA reductase inhibitor.

The first and second pharmaceutical compositions preferably are different, i.e., preferably are administered as separate dosage units (e.g., tablets). The container in the kit also preferably provides means for separating the first and second pharmaceutical compositions. For example, the container may be a divided bottle or a divided foil packet (e.g., blister pack). Typically the kit comprises directions for the administration of the separate components. Optionally, a dispenser designed to dispense the daily doses one at a time in the order of their intended use is provided. Preferably, the dispenser is equipped with a memory aid, so as to further facilitate compliance with the regimen. For example, the memory aid may be a mechanical counter that indicates the number of daily doses that has been dispensed. Another example of such a memory-aid is a battery-powered microchip memory coupled with a liquid crystal readout, or audible reminder signal which, for example, reads out the date that the last daily dose has been taken and/or reminds one when the next dose is to be taken.

The method disclosed is for the treatment or prophylaxis of a cardiovascular disorder in a patient. The method comprises treating the patient with a therapeutically effective amount of a combination of (a) Compound I, and (b) at least one HMG CoA reductase inhibitor.

The combination, pharmaceutical composition, package, kit, and method of the invention desirably involve a therapeutically effective amount of the combination of (a) Compound I and (b) an HMG CoA reductase inhibitor. Preferably, at least one of the compounds is in a therapeutically effective amount. More preferably, each of (a) Compound I and (b) an HMG CoA reductase inhibitor, is in a therapeutically effective amount.

The amount of Compound I typically administered to a patient will be 100 mg to 1800 mg per day, preferably 300 mg to 900 mg per day, more preferably 300 mg, 600 mg, or 900 mg per day, and most preferably 600 mg per day. If desired, the daily dose of Compound I can be administered to a patient once per day or alternatively as two, three, four, or more sub-doses administered separately at appropriate intervals throughout the day, optionally, in unit dosage forms. Each such sub-dose preferably contains a therapeutically effective amount of Compound I. The combination, pharmaceutical composition, package, and kit can contain any suitable amount of Compound I and typically will contain the aforementioned total amount or sub-dose to be administered to a patient per day. In accordance with the inventive method, Compound I may be administered with or without food. When administered at multiple times throughout the day, each individual dose desirably contains a therapeutically effective amount of Compound I. In a preferred embodiment of the invention, Compound I is administered with food, e.g., once per day with food. As used herein, the term "with food" is defined to mean, in general, the condition of having consumed food during the period between from about 1 hour prior to the administration of the compound to about 2 hours after the administration of the compound. Preferably, the food is a solid food with sufficient bulk and fat content that it is not rapidly dissolved and absorbed in the stomach. More preferably, the food is a meal, such as breakfast, lunch, or dinner.

The amount of an HMG CoA reductase inhibitor typically administered to the patient will be 1 mg to 100 mg per day, more preferably 1 mg to 80 mg per day (e.g, 5 mg to 80 mg per day). If desired, the daily dose of the HMG CoA reductase inhibitor can be administered to a patient once per day or alternatively as two, three, four, or more sub-doses administered separately at appropriate intervals throughout the day, optionally, in unit dosage forms. Each such sub-dose preferably contains a therapeutically effective amount of the HMG CoA reductase inhibitor. The combination, pharmaceutical composition, package, and kit can contain any suitable amount of the HMG CoA reductase inhibitor, and typically will contain the aforementioned total amount or sub-dose to be administered to a patient per day. In accordance with the inventive method, the HMG CoA reductase inhibitor may be administered with or without food. When administered at multiple times throughout the day, each individual dose desirably contains a therapeutically effective amount of the HMG CoA reductase inhibitor. In a preferred embodiment of the invention, the HMG CoA reductase inhibitor is administered once per day at bedtime.

Compound I, and at least one HMG CoA reductase inhibitor can be formulated in one dosage unit or as separate dosage units (i.e., different pharmaceutical compositions). If the compounds are in separate dosage units, Compound I, and the HMG CoA reductase inhibitor can be administered at the same time, substantially the same time, or at separate times throughout the day. In one embodiment of the invention, Compound I, is administered with food, and the HMG CoA reductase inhibitor is administered in the evening, e.g., before bedtime.

As used herein, the term "unit dosage form" is defined to refer to the form in which Compound I, or a prodrug of the active form of Compound I, and/or the HMG CoA reductase inhibitor is administered to the patient. Specifically, the unit dosage form can be, for example, a pill, capsule, or tablet. Preferably, the unit dosage form is a tablet. The typical amount of Compound I, in a unit dosage form in the context of the invention is 100 mg to 1800 mg, preferably 100 mg to 900 mg (e.g., 100 mg to 300 mg). In a preferred embodiment of the invention, the unit dosage form comprises 300 mg of Compound I, and is in the form of a tablet. Preferably, one, two, or three tablets, each of which comprises 300 mg of Compound I, are administered to the patient once per day (i.e., a total dose per day of 300 mg, 600 mg, or 900 mg, respectively). The typical amount of the HMG CoA reductase inhibitor in a unit dosage form in the context of the invention is 1 mg to 100 mg, preferably 1 to 80 mg (e.g., 5 mg to 80 mg).

The typical amount of atorvastatin calcium (e.g., as atorvastatin calcium (trihydrate)) in a unit dosage form is 10 mg, 20 mg, 40 mg, or 80 mg (see, e.g., Physicians' Desk Reference, 57th ed., Thomson PDR, 2003). A suitable dose of atorvastatin calcium (e.g., as atorvastatin calcium (trihydrate)) is 10 mg to 80 mg per day. Atorvastatin calcium (e.g., as atorvastatin calcium (trihydrate)) may be administered at any time during the day, preferably as a single dose, with or without food. Most preferably, 10 mg to 80 mg per day of atorvastatin calcium (e.g., as atorvastatin calcium (trihydrate)) is orally administered to a patient in combination with 300 mg to 900 mg (e.g., 300 mg or 600 mg) per day of Compound I.

The typical amount of pravastatin sodium in a unit dosage form is 10 mg, 20 mg, or 40 mg (see, e.g., Physicians' Desk Reference, 57th ed., Thomson PDR, 2003). A suitable total daily dose of pravastatin sodium is 20 mg to 80 mg per day. Pravastatin sodium may be administered at any time during the day, preferably as a single dose in the evening. More preferably, 20 mg to 80 mg per day of pravastatin sodium is orally administered to a patient in combination with 300 mg to 900 mg (e.g., 300 mg or 600 mg) per day of Compound I. Most preferably, 30 mg to 50 mg (e.g., 40 mg) per day of pravastatin sodium is orally administered to a patient with 300 mg to 900 mg (e.g., 300 mg or 600 mg) per day of Compound L

The typical amount of fluvastatin sodium in a unit dosage form is 20 mg, 40 mg, or 80 mg (see, e.g., Physicians' Desk Reference, 57th ed., Thomson PDR, 2003). A suitable total daily dose of fluvastatin sodium is 20 mg to 80 mg per day. Fluvastatin sodium may be administered at any time during the day, preferably as a single dose, with or without food. Most preferably, 20 mg to 80 mg per day of fluvastatin sodium is orally administered to a patient in combination with 300 mg to 900 mg (e.g., 300 mg or 600 mg) per day of Compound L

The typical amount of simvastatin in a unit dosage form is 5 mg, 10 mg, 20 mg, 40 mg, or 80 mg (see, e.g., Physicians' Desk Reference, 57th ed., Thomson PDR, 2003). A suitable total daily dose of simvastatin is 5 mg to 80 mg per day. Simvastatin may be administered at any time during the day, preferably in a single dose at bedtime. Most preferably, 5 mg to 80 mg per day of simvastatin is orally administered to a patient in combination with 300 mg to 900 mg (e.g., 300 mg or 600 mg) per day of Compound I.

The typical amount of lovastatin in a unit dosage form is 10 mg, 20 mg, 40 mg, or 60 mg (see, e.g., Physicians' Desk Reference, 57th ed., Thomson PDR, 2003). A suitable total daily dose lovastatin is 10 mg to 80 mg per day. Lovastatin may be administered at any time during the day, preferably as a single dose in the evening with a meal. Most preferably, 10 mg to 80 mg per day of lovastatin is orally administered to a patient in combination with 300 mg to 900 mg (e.g., 300 mg or 600 mg) per day of Compound I.

The typical amount of rosuvastatin calcium in a unit dosage form is 10 mg, 20 mg, 30 mg, 40 mg, or 80 mg. A suitable total daily dose of rosuvastatin calcium is 10 mg to 80 mg per day. Rosuvastatin calcium may be administered at any time during the day, preferably as a single dose, with or without food. Most preferably, 10 mg to 80 mg (e.g., 10 mg to 40 mg) per day of rosuvastatin calcium is orally administered to a patient in combination with 300 mg to 900 mg (e.g., 300 mg or 600 mg) per day of Compound I.

The typical amount of pitavastatin calcium in a unit dosage form is 1 mg, 2 mg, 4 mg, 8 mg, or 10 mg. A suitable total daily dose of pitavastatin calcium is 1 mg to 80 mg per day. Most preferably, 1 mg to 80 mg (e.g., 1 mg to 20 mg) per day of pitavastatin is orally administered to a patient in combination with 300 mg to 900 mg (e.g., 300 mg or 600 mg) per day of Compound I.

Table 1 sets forth contemplated amounts of each of Compound I and an HMG CoA reductase inhibitor in the combination, pharmaceutical composition, package, and kit of the invention, as well as to be administered daily to a patient in the method of the invention. The indication "X" in Table 1 denotes the presence and/or daily administration of the indicated amount of the indicated HMG CoA reductase inhibitor in combination with the indicated amount of Compound L Other amounts of Compound I and HMG CoA reductase inhibitors, although not reflected in Table 1, also are encompassed by the invention.

**Table 1: Combinations of Compound I and HMG CoA Reductase Inhibitor**

| | ***S*-[2-([[1-(2-ethylbutyl)cyclohexyl]carbonyl]amino)phenyl]2-methylpropanethioate** | | | | | | |
|---|---|---|---|---|---|---|---|
| **HMG CoA reductase inhibitor** | 300 mg | 400 mg | 500 mg | 600 mg | 700 mg | 800 mg | 900 mg |
| Atorvastatin calcium (e.g., as atorvastatin calcium (trihydrate)) | | | | | | | |
| 10 mg | X | X | X | X | X | X | X |
| 20 mg | X | X | X | X | X | X | X |
| 30 mg | X | X | X | X | X | X | X |
| 40 mg | X | X | X | X | X | X | X |
| 50 mg | X | X | X | X | X | X | X |
| 60 mg | X | X | X | X | X | X | X |
| 70 mg | X | X | X | X | X | X | X |
| 80 mg | X | X | X | X | X | X | X |

| Pravastatin sodium | | | | | | | |
|---|---|---|---|---|---|---|---|
| 10 mg | X | X | X | X | X | X | X |
| 20 mg | X | X | X | X | X | X | X |
| 30 mg | X | X | X | X | X | X | X |
| 40 mg | X | X | X | X | X | X | X |
| 50 mg | X | X | X | X | X | X | X |
| 60 mg | X | X | X | X | X | X | X |
| 70 mg | X | X | X | X | X | X | X |
| 80 mg | X | X | X | X | X | X | X |

| Fluvastatin sodium | | | | | | | |
|---|---|---|---|---|---|---|---|
| 20 mg | X | X | X | X | X | X | X |
| 40 mg | X | X | X | X | X | X | X |
| 60 mg | X | X | X | X | X | X | X |
| 80 mg | X | X | X | X | X | X | X |

| Simvastatin | | | | | | | |
|---|---|---|---|---|---|---|---|
| 5 mg | X | X | X | X | X | X | X |
| 10 mg | X | X | X | X | X | X | X |
| 20 mg | X | X | X | X | X | X | X |
| 30 mg | X | X | X | X | X | X | X |
| 40 mg | X | X | X | X | X | X | X |
| 50 mg | X | X | X | X | X | X | X |
| 60 mg | X | X | X | X | X | X | X |
| 70 mg | X | X | X | X | X | X | X |
| 80 mg | X | X | X | X | X | X | X |

| Lovastatin | | | | | | | |
|---|---|---|---|---|---|---|---|
| 10 mg | X | X | X | X | X | X | X |
| 20 mg | X | X | X | X | X | X | X |
| 30 mg | X | X | X | X | X | X | X |
| 40 mg | X | X | X | X | X | X | X |
| 50 mg | X | X | X | X | X | X | X |
| 60 mg | X | X | X | X | X | X | X |
| 70 mg | X | X | X | X | X | X | X |
| 80 mg | X | X | X | X | X | X | X |

| Rosuvastatin calcium | | | | | | | |
|---|---|---|---|---|---|---|---|
| 10 mg | X | X | X | X | X | X | X |
| 20 mg | X | X | X | X | X | X | X |
| 30 mg | X | X | X | X | X | X | X |
| 40 mg | X | X | X | X | X | X | X |
| 50 mg | X | X | X | X | X | X | X |
| 60 mg | X | X | X | X | X | X | X |
| 70 mg | X | X | X | X | X | X | X |
| 80 mg | X | X | X | X | X | X | X |

| Pitavastatin calcium | | | | | | | |
|---|---|---|---|---|---|---|---|
| 1 mg | X | X | X | X | X | X | X |
| 2 mg | X | X | X | X | X | X | X |
| 4 mg | X | X | X | X | X | X | X |
| 8 mg | X | X | X | X | X | X | X |
| 10 mg | X | X | X | X | X | X | X |
| 16 mg | X | X | X | X | X | X | X |
| 20 mg | X | X | X | X | X | X | X |
| 24 mg | X | X | X | X | X | X | X |
| 30 mg | X | X | X | X | X | X | X |
| 32 mg | X | X | X | X | X | X | X |
| 40 mg | X | X | X | X | X | X | X |
| 50 mg | X | X | X | X | X | X | X |
| 60 mg | X | X | X | X | X | X | X |
| 64 mg | X | X | X | X | X | X | X |
| 70 mg | X | X | X | X | X | X | X |
| 80 mg | X | X | X | X | X | X | X |

The combination of (a) Compound I and (b) at least one HMG CoA reductase inhibitor may be administered for therapy or prophylaxis to a patient in any conventional manner. While it is possible for the active ingredients, i.e., Compound I, and the HMG CoA reductase inhibitor, to be administered as the raw chemicals, preferably each active ingredient is administered as a pharmaceutical composition. Such a pharmaceutical composition comprises, for example, Compound I, and/or the HMG CoA reductase inhibitor, with one or more pharmaceutically acceptable carriers or excipients and optionally other therapeutic agents and/or components. The carriers or excipients must be acceptable in the sense of being compatible with the other ingredients and not deleterious to the recipient thereof. Examples of carriers or excipients for oral administration include cornstarch, lactose, magnesium stearate, talc, microcrystalline cellulose, stearic acid, povidone, crospovidone, dibasic calcium phosphate, sodium starch glycolate, hydroxypropyl cellulose (e.g., low substituted hydroxypropyl cellulose), hydroxypropylmethyl cellulose (e.g., hydroxypropylmethyl cellulose 2910), and sodium lauryl sulfate.

The pharmaceutical composition can be prepared by any suitable method, such as those methods well known in the art of pharmacy, for example, methods such as those described in Gennaro et al., Remington's Pharmaceutical Sciences (18th ed., Mack Publishing Co., 1990), especially Part 8: Pharmaceutical Preparations and their Manufacture. Such methods include the step of bringing into association one or more of the active compounds, e.g., Compound I and/or an HMG CoA reductase inhibitor, with the carrier or excipient and optionally one or more accessory ingredients. Such accessory ingredients include those conventional in the art, such as, fillers, binders, diluents, disintegrants, lubricants, colorants, flavoring agents, and wetting agents.

The pharmaceutical composition can provide controlled, slow release, or sustained release of one or more of the active compounds, e.g., Compound I and/or the HMG CoA reductase inhibitor, over a predetermined period of time. The controlled, slow release, or sustained release of the therapeutic compound can provide for a concentration of one or more of the active forms of the active compounds, e.g., the active form of Compound I and/or the HMG CoA reductase inhibitor, to be maintained in the bloodstream of the patient for a longer period of time than with conventional formulations. Such a pharmaceutical composition can be a coated tablet, pellet, or capsule, as well as a dispersion of one or more of the active compounds in a medium that is insoluble in physiologic fluids or where the release of the active compound(s) follows degradation of the pharmaceutical composition due to mechanical, chemical, or enzymatic activity.

The pharmaceutical composition in the context of the invention can be, for example, in the form of a pill, capsule, or tablet, each containing a predetermined amount of one or more of the active compounds, e.g., Compound I and/or the HMG CoA reductase inhibitor, and preferably coated for ease of swallowing, in the form of a powder or granules, or in the form of a solution or suspension. Preferably, the pharmaceutical composition is in the form of a tablet comprising one or more of the active compounds, e.g., Compound I and/or an HMG CoA reductase inhibitor and the components of the tablet utilized and described in the Examples herein. For oral administration, fine powders or granules may contain diluting, dispersing, and or surface active agents and may be present, for example, in water or in a syrup, in capsules or sachets in the dry state, or in a nonaqueous solution or suspension wherein suspending agents may be included, or in tablets wherein binders and lubricants may be included. Components such as sweeteners, flavoring agents, preservatives (e.g., antimicrobial preservatives), suspending agents, thickening agents, and/or emulsifying agents also may be present in the pharmaceutical composition. When administered in the form of a liquid solution or suspension, the formulation can contain one or more of the active compounds, e.g., Compound I and/or an HMG CoA reductase inhibitor, and purified water. Optional components in the liquid solution or suspension include suitable sweeteners, flavoring agents, preservatives (e.g., antimicrobial preservatives), buffering agents, solvents, and mixtures thereof. A component of the formulation may serve more than one function. For example, a suitable buffering agent also may act as a flavoring agent as well as a sweetener.

Suitable sweeteners include, for example, saccharin sodium, sucrose, and mannitol. A mixture of two or more sweeteners optionally may be used. The sweetener or mixtures thereof are typically present in an amount of from about 0.001% to about 70% by weight of the total composition. Suitable flavoring agents may be present in the pharmaceutical composition to provide a cherry flavor, cotton candy flavor, or other suitable flavor to make the pharmaceutical composition easier for a patient to ingest. The flavoring agent or mixtures thereof are typically present in an amount of about 0.0001% to about 5% by weight of the total composition.

Suitable preservatives include, for example, methylparaben, propylparaben, sodium benzoate, and benzalkonium chloride. A mixture of two or more preservatives optionally may be used. The preservative or mixtures thereof are typically present in an amount of about 0.0001% to about 2% by weight of the total composition.

Suitable buffering agents include, for example, citric acid, sodium citrate, phosphoric acid, potassium phosphate, and various other acids and salts. A mixture of two or more buffering agents optionally may be used. The buffering agent or mixtures thereof are typically present in an amount of about 0.001 % to about 4% by weight of the total composition.

Suitable solvents for a liquid solution or suspension include, for example, sorbital, glycerin, propylene glycol, and water. A mixture of two or more solvents optionally may be used. The solvent or solvent system is typically present in an amount of about 1% to about 90% by weight of the total composition.

Oral delivery methods are often limited by chemical and physical barriers imposed by the body, such as the varying pH in the gastrointestinal tract, exposure to enzymes, and the impermeability of the gastrointestinal membranes. The oral administration of the pharmaceutical composition may also include the co-administration of adjuvants. For example, nonionic surfactants such as polyoxyethylene oleyl ether and n-hexadecyl polyethylene ether can be administered with or incorporated into the pharmaceutical composition to artificially increase the permeability of the intestinal walls. Enzymatic inhibitors also can be administered with or incorporated into the pharmaceutical composition.

The active compounds of the combination, pharmaceutical composition, package, kit, and method of the invention, when administered to a patient on a daily basis, desirably result (e.g., at 2 weeks, 4 weeks, 8 weeks, 12 weeks and/or six months post-treatment initiation) in one or more of the following conditions in the patient: (a) an inhibition of cholesteryl ester transfer protein (CETP) activity in the patient relative to pretreatment CETP activity, (b) an increase in high density lipoprotein cholesterol (HDL-C) level in the patient relative to pretreatment HDL-C level, (c) a decrease in low density lipoprotein cholesterol (LDL-C) level in the patient relative to pretreatment LDL-C level, (d) a decrease in the ratio of total cholesterol to HDL-C level (TC/HDL-C) in the patient relative to pretreatment TC/HDL-C, and/or (e) a decrease in the ratio of LDL-C level to HDL-C level (LDL-C/HDL-C) relative to pretreatment LDL-C/HDL-C. The term "pretreatment" refers to the time prior (desirably immediately prior) to administration of the active compounds of the combination, pharmaceutical composition, package, kit, and method of the invention to the patient. The desired extent of changes in each of the foregoing conditions in the patient relative to pretreatment are recited below. Preferably, the active compounds of the combination, pharmaceutical composition, package, kit, and method of the invention, when administered to a patient, result in two or more (e.g., two, three, four, or five) of the foregoing conditions in the patient (e.g., at 2 weeks, 4 weeks, 8 weeks, 12 weeks and/or six months post-treatment initiation). Most preferably, the active compounds of the combination, pharmaceutical composition, package, kit, and method of the invention, when administered to a patient, result in all five of the foregoing conditions in the patient (e.g., at 2 weeks, 4 weeks, 8 weeks, 12 weeks and/or six months post-treatment initiation).

CETP activity is measured essentially as described in Tollefson et al., Methods Enzymol., 129, 797-816 (1986), and Kato et al., J. Biol. Chem., 264, 4082-4087 (1989). Preferably, the CETP activity at 2 weeks (or 4 weeks, 8 weeks, 12 weeks, or six months) post-treatment initiation is decreased by about 5% or more relative to CETP activity pretreatment (e.g., about 7.5% or more, about 10% or more, about 15% or more, about 20% or more, about 25% or more, about 30% or more, about 35% or more, about 40% or more, about 45% or more, about 50% or more, about 55% or more, about 60% or more, or about 65% or more).

The HDL-C level is measured using standard techniques known in the art. Preferably, the HDL-C level at 2 weeks (or 4 weeks, 8 weeks, 12 weeks, or six months) post-treatment initiation is increased by about 5% or more relative to pretreatment HDL-C level (e.g., about 7.5% or more, about 10% or more, about 12.5% or more, about 15% or more, about 17.5% or more, about 20% or more, about 22.5% or more, about 25% or more, about 27.5% or more, about 30% or more, about 32.5% or more, about 35% or more, about 37.5% or more, about 40% or more, about 42.5% or more, about 45% or more, about 47.5% or more, about 50% or more, about 52.5% or more, about 55% or more, about 57.5% or more, or about 60% or more).

The LDL-C level is measured using standard techniques known in the art. Preferably, the LDL-C level at 2 weeks (or 4 weeks, 8 weeks, 12 weeks, or six months) post-treatment initiation is decreased by about 5% or more relative to pretreatment LDL-C level (e.g., about 7.5% or more, about 10% or more, about 12.5% or more, about 15% or more, about 17.5% or more, about 20% or more, about 22.5% or more, about 25% or more, about 27.5% or more, about 30% or more, about 32.5% or more, about 35% or more, about 37.5% or more, about 40% or more, about 42.5% or more, about 45% or more, about 47.5% or more, about 50% or more, about 52.5% or more, about 55% or more, about 57.5% or more, or about 60% or more).

Total cholesterol (TC) is determined using standard techniques known in the art. Preferably, the TC/HDL-C ratio at 2 weeks (or 4 weeks, 8 weeks, 12 weeks, or six months) post-treatment initiation is decreased by about 5% or more relative to the pretreatment TC/HDL-C ratio (e.g., about 7.5% or more, about 10% or more, about 12.5% or more, about 15% or more, about 17.5% or more, about 20% or more, about 22.5% or more, about 25% or more, about 27.5% or more, about 30% or more, about 32.5% or more, or about 35% or more).

With respect to the ratio of LDL-C level to HDL-C level (LDL-C/HDL-C), LDL-C/HDL-C at 2 weeks (or 4 weeks, 8 weeks, 12 weeks, or six months) post-treatment initiation preferably is decreased by about 5% or more relative to pretreatment LDL-C/HDL-C (e.g., about 7.5% or more, about 10% or more, about 12.5% or more, about 15% or more, about 17.5% or more, about 20% or more, about 22.5% or more, about 25% or more, about 27.5% or more, about 30% or more, about 32.5% or more, or about 35% or more).

The patient that is administered a combination of Compound I and an HMG CoA reductase inhibitor can be any patient in need of treatment or prevention of a cardiovascular disorder. For example, the patient can exhibit an HDL-C level of about 60 mg/dL or less (e.g., about 50 mg/dL or less, or about 40 mg/dL or less) prior to initiating treatment or prophylaxis with the combination.

Alternatively or additionally, the patient can have a medical history of, or be currently diagnosed with, coronary heart disease or coronary heart disease risk equivalent as defined by at least one of the following: atherosclerotic disease (e.g., peripheral arterial disease, abdominal aortic aneurysm, or symptomatic carotid artery disease); type II diabetes (wherein the patient requires a lipid lowering agent to treat hypercholesterolemia and/or hyperbetalipoproteinemia); and Framingham 10-years coronary heart disease risk about 20% or more.

Alternatively or additionally, the patient can exhibit at least one of the following risk factors: cigarette smoking, hypertension (blood pressure (BP) ≥ 140/90 mm Hg or on hypertension medication); family history of premature coronary heart disease (coronary heart disease in male first degree relative (parent, sibling, or offspring) less than 55 years in age; coronary heart disease in female first degree relative less than 65 years in age); and age (men ≥ 45 years; women ≥ 55 years).

Alternatively or additionally, the patient can have a Framingham 10-years coronary heart disease risk of about 10% or less. More preferably, the patient has a Framingham 10-years coronary heart disease risk of from about 10% to about 20%. Most preferably, the patient has a Framingham 10-years coronary heart disease risk of about 20% or more. Those skilled in the art are familiar with how to determine a Framingham 10-years coronary heart disease risk value.

The patient preferably exhibits one or more risk factors from at least one of the above risk categories before initiating treatment or prophylaxis with the combination. Preferably, the patient exhibits one or more risk factors from at least two of the above risk categories, and more preferably, the patient exhibits one or more risk factors from at least three of above risk categories. Desirably, the patient exhibits one or more risk factors from each of the four above risk categories.

The following examples further illustrate the invention but, of course, should not be construed as in any way limiting its scope.

### EXAMPLE 1

This example describes a multi-center, randomized, double-blind, placebo-controlled, parallel group study designed to evaluate the therapeutic effect of the combination of *S*-[2-([[1-(2-ethylbutyl)cyclohexyl]carbonyl]amino)phenyl]2-methylpropanethioate (Compound I) and pravastatin sodium in patients with hyperlipidemia.

Approximately 200 patients with type II hyperlipidemia were enrolled in the study to ensure that about 150 patients (about 50 patients per treatment group) completed the study. The patients met the following criteria:
(i) LDL-C > 4.0 mmol/L (160 mg/dL),
(ii) HDL-C < 1.6 mmol/L (60 mg/dL),
(iii) triglycerides < 4.5 mmol/L (400 mg/dL), and
(iv) age 18-65 years.

The total study duration was 16 weeks, which consisted of three periods. During Period 1, the first 8 weeks of the study, 40 mg of pravastatin sodium was taken once daily at bedtime by all enrolled patients. Patients were then randomized to receive placebo or 300 mg or 600 mg of Compound I once daily following breakfast for Period 2, the next 4-week period, in addition to the continued pravastatin sodium treatment. During Period 3, the last 4 weeks of the study, pravastatin sodium was administered alone.

Each pravastatin sodium tablet comprised 40 mg of pravastatin sodium that was provided as a branded commercial product (Selektine^{™}, Bristol-Myers Squibb B.V.). Each Compound I tablet contained 300 mg of Compound I and was supplied as a white oval tablet that is identical in appearance to the placebo tablet. The uncoated white tablets comprising Compound I were prepared using standard tableting procedures. The tablets comprised 300 mg of Compound I, 18 mg of hydroxypropylmethyl cellulose 2910 as a binder, 18 mg of talc and 1.2 mg of magnesium stearate as lubricants, and 119.8 mg of crospovidone and 90 mg of low-substituted hydroxypropyl cellulose as disintegrants.

All patients received 2 uncoated white tablets, such that each patient received either (a) one 300 mg Compound I tablet and one placebo tablet, (b) two 300 mg Compound I tablets, or (c) two placebo tablets during Period 2.

Blood samples were taken at Visit 1 (-10 weeks relative to commencement of treatment with Compound I or placebo) to determine eligibility, and at Visit 2 (-8 weeks; commencement of treatment with pravastatin sodium), Visit 3 (-2 weeks), Visit 4 (commencement of treatment with Compound I or placebo), Visit 5 (+2 weeks after commencement of treatment with Compound I or placebo), Visit 6 (+4 weeks; end of treatment with Compound I or placebo), and Visit 7 (+8 weeks; end of treatment with pravastatin sodium). Each blood sample was tested for levels of lipid parameters (e.g., total cholesterol, triglycerides, HDL-C, LDL-C), CETP activity and mass, the plasma concentration of Compound I (trough level), as well as laboratory safety parameters (e.g., urinalysis, biochemistry, and hematology).

The procedure for determining CETP activity was substantially similar to the procedures described in Tollefson et al., Methods Enzymol., 129, 797-816 (1986), and Kato et al., J. Biol. Chem., 264, 4082-4087 (1989).

The plasma concentration of the active form of Compound I was determined by the following assay. Plasma samples were isolated from patients treated with Compound I. The plasma samples were treated with sodium hydroxide to convert active forms of Compound I in the plasma to the thiol form (i.e., Compound II). The plasma sample next was treated with dithiothreitol (DTT) to prevent the oxidation of thiol groups (i.e., to maintain thiol groups in a reduced state). N-ethylmaleimide (NEM) was added to stabilize the thiol form (i.e., Compound II) by, it is believed, blocking the free sulfhydryl group by the derivatization to an NEM-adduct. The sample then was analyzed using High Performance Liquid Chromatography (HPLC). Finally, the results of the HPLC analysis of the plasma sample were compared to a known standard to determine the plasma concentration of the active form of Compound L The standard of known concentration was prepared essentially as described above, with the exception that human plasma was isolated from humans who were not treated with Compound I. These "blank plasma" samples were combined with a known amount of Compound I.

Efficacy analyses included both descriptive summary and statistical modeling, such as analysis of variance (ANOVA) or analysis of covariance (ANCOVA). The descriptive summary included frequencies and percentages for categorical variables and statistics such as the number of available observations, mean, median, standard deviation, minimum, and maximum for all variables.

To determine the effect of the administration of Compound I on a patient receiving HMG CoA reductase inhibitor therapy, the mean values of HDL-C, LDL-C, TC/HDL-C ratio, LDL-C/HDL-C ratio, and CETP activity before administration of Compound I (average of values at Visits 3 and 4) and after the administration of Compound I (Visit 5 or 6) were compared. An ANOVA model containing treatment and center effects was used. The inferential tests of superiority were carried out at a 2.5% level of significance (1-sided) with the following step down procedure:
1. a superiority test of the high dose group (receiving 600 mg of Compound I) versus placebo, and followed with
2. a superiority test of the low dose group (receiving 300 mg of Compound I) versus placebo if only the superiority of the high dose group was established in step 1; otherwise, no further tests were carried out.

Further analysis of the primary efficacy endpoints involved the inclusion of treatment-center interaction effect in the analysis of variance mode, the inclusion of treatment effect and the respective baseline value as the covariate in the ANCOVA model, and the addition of treatment-covariate interaction effect in the ANCOVA model. If the interaction effect was qualitative (treatment differences were not all in the same direction) and was significant at the significance level of 0.2 (2-sided), subgroup analyses were conducted. The primary analysis was calculated based on absolute change data instead of percentage changes. If the assumptions for ANOVA/ANCOVA were violated, the primary endpoints were analyzed on a log scale.

Table 2 describes the percent change in HDL-C, LDL-C, TC/HDL-C ratio, LDL-C/HDL-C ratio, and CETP activity from the values of patients receiving pravastatin sodium therapy (average of Visit 3 and 4 values) to the values following administration of Compound I or placebo and pravastatin sodium (Visit 5 or 6). P values were determined by comparing the groups administered Compound I to the placebo group using an ANOVA model with percent change as the dependent variable and treatment and center as the fixed effects. The data in Table 2 are from patients that completed the treatment per protocol.

**Table 2: Mean (S.D.) Percent Changes of Parameters in Patients Receiving Pravastatin Sodium Therapy after the Administration of Compound I or Placebo**

| | **Percent Changes from Levels before Administration of Compound I (standard deviation)** | | | | |
|---|---|---|---|---|---|
| **Treatment Protocol** | **HDL-C** | **LDL-C** | **TC/ LDL-C ratio** | **LDL-C/ HDL-C ratio** | **CETP activity** |
| **Placebo (n=40)** | 0.41 (10.697) | 1.44 (14.949) | 1.18 (11.035) | 3.26 (17.241) | 2.44 (8.782) |
| **300 mg Compound I (n=45)** | 12.96* (13.009) | 1.57 (14.057) | -8.09^{†} (14.279) | -10.31^{†} (19.514) | -18.10* (13.984) |
| **600 mg Compound I (n=42)** | 28.43 * (16.324) | -7.91^{‡} (15.108) | -18.72* (13.251) | -26.03* (17.143) | -31.62* (11.627) |

| | | | | | |
|---|---|---|---|---|---|
| * p < 0.001 ^{‡}p ≤ 0.01 ^{†}p < 0.005 | | | | | |

As depicted in Table 2, the administration of Compound I to patients receiving HMG CoA reductase inhibitor therapy significantly affects the HDL-C level, CETP activity, and the ratios of TC/HDL-C and LDL-C/HDL-C of the patients. As expected, there was no significant change in any of these parameters in the group of patients receiving placebo rather than Compound I.

The data set forth in Table 2 demonstrate that the daily administration of Compound I to patients receiving pravastatin sodium therapy can achieve an increase in HDL-C levels of at least about 5% relative to levels achieved by the administration of pravastatin sodium alone. For example, after the administration of Compound I, HDL-C levels were increased by about 13% and about 28% in the 300 mg and 600 mg treatment groups, respectively, relative to levels achieved by the administration of pravastatin sodium alone.

The data set forth in Table 2 illustrate that the TC/HDL-C ratio can be decreased by at least about 5% following daily administration of Compound I to patients receiving pravastatin sodium therapy relative to levels achieved by the administration of pravastatin sodium alone. For example, after the administration of Compound I, the TC/HDL-C ratios were decreased by about 8% and about 19% in the 300 mg and 600 mg treatment groups, respectively, relative to levels achieved by the administration of pravastatin sodium alone.

The data set forth in Table 2 also demonstrate that the LDL-C/HDL-C ratio can be decreased by at least about 5% following daily administration of Compound I to patients receiving pravastatin sodium therapy relative to levels achieved by the administration of pravastatin sodium alone. For example, after the administration of Compound I, the LDL-C/HDL-C ratios were decreased by about 10% and about 26% in the 300 mg and 600 mg treatment groups, respectively, relative to levels achieved by the administration of pravastatin sodium alone.

Additionally, the data set forth in Table 2 demonstrate that the administration of Compound I to patients receiving pravastatin sodium therapy can achieve a decrease in CETP activity of at least about 5% relative to levels achieved by the administration of pravastatin sodium alone. For example, after administration of Compound I, CETP activity decreased by about 18% and about 32% in the 300 mg and 600 mg treatment groups, respectively, relative to levels achieved by the administration of pravastatin sodium alone.

Moreover, as is apparent from the data set forth in Table 2, the administration of Compound I to patients receiving pravastatin sodium therapy can achieve a decrease in LDL-C levels of at least 5% relative to levels achieved by the administration of pravastatin sodium alone. For example, after the administration of Compound I, LDL-C levels decreased by about 8% in the 600 mg treatment group relative to the level achieved by the administration of pravastatin sodium alone.

### EXAMPLE 2

This example describes a multi-center, randomized, double-blind, placebo-controlled, parallel group study designed to evaluate the therapeutic effect of the combination of *S*-[2-([[1-(2-ethylbutyl)cyclohexyl]carbonyl]amino)phenyl] 2-methylpropanethioate (Compound I) and atorvastatin calcium (trihydrate) in patients with low HDL levels.

Approximately 80 patients are enrolled in the study who meet the following criteria:
(i) low HDL levels (documented HDL-C levels ≤ 1.0 mmol (40 mg/dL) and triglycerides (TG) ≤ 4.5 mmol/L (400 mg/dL)),
(ii) age 18 to 70 years, and
(iii) a medical history of, or currently diagnosed with, Coronary Heart Disease (CHD) or CHD risk equivalent as defined by at least one of the following: atherosclerotic disease (peripheral arterial disease, abdominal aortic aneurysm, or symptomatic carotid artery disease); type II diabetes (requiring a lipid lowering agent); and Framingham 10-years CHD risk more than 20%.

The total study duration is 16 weeks, which consists of three periods. During Period 1, the first 8 weeks of the study, 20 mg of atorvastatin (as atorvastin calcium trihydrate) is taken once daily immediately following breakfast by all enrolled patients. Patients are then randomized to receive placebo or 600 mg of Compound I (approximately 40 patients per each of the two groups) once daily following breakfast for Period 2, the next 4-week period, in addition to the continued atorvastatin calcium (trihydrate) treatment. During Period 3, the last 4 weeks of the study, atorvastatin calcium (trihydrate) is administered alone.

Each atorvastatin tablet comprises 20 mg of atorvastatin (as atorvastatin calcium trihydrate) that is provided as a branded commercial product (Sortis^{™}, Parke-Davis GmbH). The tablets comprising Compound I, and the preparation thereof, are described in Example 1. All patients receive 2 uncoated white tablets, such that each patient receives either (a) two 300 mg Compound I tablets or (b) two placebo tablets during Period 2.

Sampling schedules and analysis parameters are commensurate with those described in Example 1.

### EXAMPLE 3

This example describes a multi-center, randomized, double-blind, placebo-controlled, parallel group study designed to evaluate the therapeutic effect of the combination of *S*-[2-([[1-(2-ethylbutyl)cyclohexyl]carbonyl]amino)phenyl]2-methylpropanethioate (Compound I) and simvastatin in patients with low HDL levels.

Approximately 80 patients are enrolled in the study who meet the following criteria:
(i) low HDL levels (documented HDL-C levels ≤ 1.0 mmol (40 mg/dL) and triglycerides (TG) ≤ 4.5 mmol/L (400 mg/dL)),
(ii) age 18 to 70 years,
(iii) a medical history of, or currently diagnosed with, Coronary Heart Disease (CHD) or CHD risk equivalent as defined by at least one of the following: atherosclerotic disease (peripheral arterial disease, abdominal aortic aneurysm, or symptomatic carotid artery disease); type II diabetes (requiring a lipid lowering agent); and Framingham 10-years CHD risk more than 20%.

The total study duration is 16 weeks, which consists of three periods. During Period 1, the first 8 weeks of the study, 40 mg of simvastatin is taken once daily immediately following breakfast by all enrolled patients. Patients are then randomized to receive placebo or 600 mg of Compound I (approximately 40 patients per each of the two groups) once daily following breakfast for Period 2, the next 4-week period, in addition to the continued simvastatin treatment. During Period 3, the last 4 weeks of the study, simvastatin is administered alone.

Each simvastatin tablet comprises 40 mg of simvastatin that is provided as a branded commercial product (Zocor^{™}, Merck Sharp & Dohme B.V.). The tablets comprising Compound I, and the preparation thereof, are described in Example 1. All patients receive 2 uncoated white tablets, such that each patient receives either (a) two 300 mg Compound I tablets or (b) two placebo tablets during Period 2.

Sampling schedules and analysis parameters are commensurate with those described in Example 1.

### EXAMPLE 4

This example describes a study to evaluate the effect of the administration of the combination of *S*-[2-([[1-(2-ethylbutyl)cyclohexyl]carbonyl]amino)phenyl] 2-methylpropanethioate (Compound I) and an HMG CoA reductase inhibitor on Japanese white rabbits receiving a high-cholesterol diet.

Prior to commencement of the study, male Japanese white rabbits (KITAYAMA LABES Co., Ltd.) received a normal diet (RC-4, manufactured by Oriental Bio-Service Inc.) (Time Period 1) and water *ad libitum.* For preliminary feeding, male Japanese white rabbits were provided with a high cholesterol diet comprising 100 g/day per rabbit of RC-4 containing 0.2% cholesterol (manufactured by Oriental Bio-Service Inc.) for 4 weeks (Time Period 2).

Blood samples were drawn from the auricular artery before feeding on the day following the last day of preliminary feeding of Time Period 2. 12-week-old male Japanese rabbits were grouped into a control group and seven test groups, with 6 rabbits per group, based on plasma parameters (HDL cholesterol content, total cholesterol content, triglyceride content) and body weight, such that no significant difference in plasma parameters or body weight was apparent among the groups. For 7 days (Time Period 3), each group was administered a high cholesterol diet comprising 100 g/day per rabbit of RC-4 containing 0.2% cholesterol and one of the following:
(1) Control (no additional components)
(2) Compound I (0.5%)
(3) Simvastatin (extracted and purified from Lipovas^{™} Tablets-5, Banyu Pharmaceutical Co.) (0.075%)
(4) Atorvastatin calcium trihydrate (extracted and purified from Lipitor^{™} Tablets-10, Pfizer Inc.) (0.075%)
(5) Rosuvastatin calcium (extracted and purified from Crestor^{™} Tablets, Astra Zeneca) (0.025%)
(6) Compound I (0.5%) + simvastatin (0.075%)
(7) Compound I (0.5%) + atorvastatin calcium trihydrate (0.075%)
(8) Compound I (0.5%) + rosuvastatin calcium (0.025%)

At 8 hours after feeding on Day 7 of Time Period 3, blood samples were taken from the auricular artery, and HDL cholesterol content and total cholesterol content in plasma were measured by standard methods. Atherogenic index was calculated as follows: [(total cholesterol content - HDL cholesterol content) / HDL cholesterol content]. Table 3 contains the percent atherogenic index of each of the test groups, wherein the percent atherogenic index of each of the test groups was calculated based on the value of the control group being 100%.

**Table 3: Atherogenic Index (%) of 12-Week Old Male Japanese Rabbits Receiving a High Cholesterol Diet**

| **Group** | **Atherogenic Index (%)** |
|---|---|
| Control | 100 |
| Compound I | 54 |
| Simvastatin | 51 |
| Atorvastatin calcium trihydrate | 49 |
| Rosuvastatin calcium | 81 |
| Compound I and simvastatin | 31 |
| Compound I and atorvastatin calcium trihydrate | 40 |
| Compound I and rosuvastatin calcium | 42 |

A similar experiment was conducted to evaluate the effect of the administration of Compound I and pravastatin sodium (Xiamen Mchem Ltd.) on 12-week old male Japanese white rabbits receiving a high-cholesterol diet. The study parameters were as described above, except each group of the animals was administered a high cholesterol diet comprising 100 g/day per rabbit of RC-4 containing 0.2% cholesterol and one of the following:
(1) Control (no additional components)
(2) Compound I (0.5%)
(3) Pravastatin sodium (0.075%)
(4) Compound I (0.5%) and pravastatin sodium (0.075%)

Table 4 contains the atherogenic index of each of the groups, wherein atherogenic index is shown based on the value of the control group as 100%.

**Table 4: Atherogenic Index (%) of 12-Week Old Male Japanese Rabbits Receiving a High Cholesterol Diet**

| **Group** | **Atherogenic Index (%)** |
|---|---|
| Control | 100 |
| Compound I | 60 |
| Pravastatin sodium | 40 |
| Compound I and pravastatin sodium | 21 |

As illustrated by the data in Tables 3 and 4, the combination of Compound I and a therapeutic agent for hyperlipidemia (e.g., an HMG CoA reductase inhibitor) decreases the atherogenic index in 12-week-old male Japanese rabbits on a high cholesterol diet as compared to control animals. Additionally, the administration of the combination of Compound I and an HMG CoA reductase inhibitor demonstrates a superior synergistic effect in amelioration of atherogenic profile (as measured by atherogenic index) when compared to animals administered Compound I or an HMG CoA reductase inhibitor alone. As such, Compound I can be administered in combination with another pharmaceutical agent, and in particular, other therapeutic agents for hyperlipidemia, atherosclerosis, coronary artery disease, obesity, diabetes, or hypertension, to increase the usefulness of these agents for treatment.

All references, including publications, patent applications, and patents, cited herein are hereby incorporated by reference to the same extent as if each reference were individually and specifically indicated to be incorporated by reference and were set forth in its entirety herein.

The use of the terms "a" and "an" and "the" and similar referents in the context of describing the invention (especially in the context of the following claims) are to be construed to cover both the singular and the plural, unless otherwise indicated herein or clearly contradicted by context. The terms "comprising," "having," "including," and "containing" are to be construed as open-ended terms (i.e., meaning "including, but not limited to,") unless otherwise noted. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (e.g., "such as") provided herein, is intended merely to better illuminate the invention and does not pose a limitation on the scope of the invention unless otherwise claimed. No language in the specification should be construed as indicating any non-claimed element as essential to the practice of the invention.

Preferred embodiments of this invention are described herein, including the best mode known to the inventors for carrying out the invention. Variations of those preferred embodiments may become apparent to those of ordinary skill in the art upon reading the foregoing description. The inventors expect skilled artisans to employ such variations as appropriate, and the inventors intend for the invention to be practiced otherwise than as specifically described herein.

Furthermore, the present invention discloses the following embodiments:
1. A combination comprising (a) *S*-[2-([[1-(2-ethylbutyl)cyclohexyl]carbonyl]amino)phenyl] 2-methylpropanethioate and (b) at least one HMG CoA reductase inhibitor.
2. The combination of any of 1, wherein the HMG CoA reductase inhibitor is selected from the group consisting of atorvastatin, pravastatin, fluvastatin, simvastatin, lovastatin, rosuvastatin, and phannaceutically acceptable salts and hydrates thereof.
3. The combination of 2, wherein the HMG CoA reductase inhibitor is atorvastatin calcium, pravastatin sodium, fluvastatin sodium, simvastatin, lovastatin, or rosuvastatin calcium.
4. The combination of any of 1, wherein the HMG CoA reductase inhibitor is pitavastatin or a pharmaceutically acceptable salt and/or hydrate thereof.
5. A pharmaceutical composition comprising (a) *S*-[2-([[1-(2-ethylbutyl)cyclohexyl]carbonyl]amino)phenyl] 2-methylpropanethioate (b) at least one HMG CoA reductase inhibitor, and (c) one or more pharmaceutically acceptable carriers.
6. The pharmaceutical composition of any of 5, wherein the HMG CoA reductase inhibitor is selected from the group consisting of atorvastatin, pravastatin, fluvastatin, simvastatin, lovastatin, rosuvastatin, and pharmaceutically acceptable salts and hydrates thereof.
7. The pharmaceutical composition of 6, wherein the HMG CoA reductase inhibitor is atorvastatin or a pharmaceutically acceptable salt and/or hydrate thereof.
8. The pharmaceutical composition of 6, wherein atorvastatin or a pharmaceutically acceptable salt and/or hydrate thereof is present in an amount of about 10 mg to about 80 mg.
9. The pharmaceutical composition of 6, wherein the HMG CoA reductase inhibitor is pravastatin or a pharmaceutically acceptable salt and/or hydrate thereof.
10. The pharmaceutical composition of 9, wherein pravastatin or a pharmaceutically acceptable salt and/or hydrate thereof is present in an amount of about 10 mg to about 40 mg.
11. The pharmaceutical composition of 10, wherein pravastatin or a pharmaceutically acceptable salt and/or hydrate thereof is present in an amount of about 40 mg.
12. The pharmaceutical composition of 6, wherein the HMG CoA reductase inhibitor is fluvastatin or a pharmaceutically acceptable salt and/or hydrate thereof.
13. The pharmaceutical composition of 12, wherein fluvastatin or a pharmaceutically acceptable salt and/or hydrate thereof is present in an amount of about 20 mg to about 80 mg.
14. The pharmaceutical composition of 6, wherein the HMG CoA reductase inhibitor is simvastatin or a pharmaceutically acceptable salt and/or hydrate thereof.
15. The pharmaceutical composition of 14, wherein simvastatin or a pharmaceutically acceptable salt and/or hydrate thereof is present in an amount of about 5 mg to about 80 mg.
16. The pharmaceutical composition of 6, wherein the HMG CoA reductase inhibitor is lovastatin or a pharmaceutically acceptable salt and/or hydrate thereof.
17. The pharmaceutical composition of 16, wherein lovastatin or a pharmaceutically acceptable salt and/or hydrate thereof is present in an amount of about 10 mg to about 60 mg.
18. The pharmaceutical composition of 6, wherein the HMG CoA reductase inhibitor is rosuvastatin or a pharmaceutically acceptable salt and/or hydrate thereof.
19. The pharmaceutical composition of 18, wherein rosuvastatin or a pharmaceutically acceptable salt and/or hydrate thereof is present in an amount of about 10 mg to about 40 mg.
20. The pharmaceutical composition of any of 5, wherein the HMG CoA reductase inhibitor is pitavastatin or a pharmaceutically acceptable salt and/or hydrate thereof.
21. The pharmaceutical composition of 20, wherein pitavastatin or a pharmaceutically acceptable salt and/or hydrate thereof is present in an amount of about 1 mg to about 80 mg.
22. The pharmaceutical composition of any of 5-21, wherein the *S*-[2-([[1-(2-ethylbutyl)cyclohexyl]carbonyl]amino)phenyl] 2-methylpropanethioate is present in an amount of about 100 mg to about 300 mg.
23. A package comprising separate dosage units, of which (a) at least one dosage unit comprises *S*-[2-([[1-(2-ethylbutyl)cyclohexyl]carbonyl]amino)phenyl] 2-methylpropanethioate, and (b) at least one other dosage unit comprises an HMG CoA reductase inhibitor.
24. The package of any of 23, wherein the HMG CoA reductase inhibitor is selected from the group consisting of atorvastatin, pravastatin, fluvastatin, simvastatin, lovastatin, rosuvastatin, and pharmaceutically acceptable salts and hydrates thereof.
25. The package of 24, wherein the HMG CoA reductase inhibitor is atorvastatin calcium, pravastatin sodium, fluvastatin sodium, simvastatin, lovastatin, or rosuvastatin calcium.
26. The package of any of 23-25, wherein the HMG CoA reductase inhibitor is present in its dosage unit in an amount of about 5 mg to about 80 mg.
27. The package of any of 23, wherein the HMG CoA reductase inhibitor is pitavastatin or a pharmaceutically acceptable salt and/or hydrate thereof.
28. The package of 27, wherein the pitavastatin is present in its dosage unit in an amount of about 1 mg to about 80 mg.
29. The package of any of 23-28, wherein the *S*-[2-([[1-(2-ethylbutyl)cyclohexyl]carbonyl]amino)phenyl] 2-methylpropanethioate is present in its dosage unit in amount of about 100 mg to about 300 mg.
30. A kit comprising (a) a first pharmaceutical composition comprising a therapeutically effective amount of (i) *S*-[2-([[1-(2-ethylbutyl)cyclohexyl]carbonyl]amino)phenyl] 2-methylpropanethioate, and (ii) a pharmaceutically acceptable carrier, (b) a second pharmaceutical composition comprising (i) at least one HMG CoA reductase inhibitor, and (ii) a pharmaceutically acceptable carrier, (c) prescribing information, and (d) a container, wherein the first and second pharmaceutical compositions can be the same or different, and wherein the prescribing information includes advice to a patient regarding co-administration of *S*-[2-([[1-(2-ethylbutyl)cyclohexyl]carbonyl]amino)phenyl] 2-methylpropanethioate, and the HMG CoA reductase inhibitor.
31. The kit of any of 30, wherein the first and second pharmaceutical compositions are different.
32. The kit of any of 30-31, wherein the therapeutically effective amount of the *S*-[2-([[1-(2-ethylbutyl)cyclohexyl]carbonyl]amino)phenyl] 2-methylpropanethioate is about 100 mg to about 300 mg.
33. The kit of any of 30-32, wherein the first and second pharmaceutical compositions are in the form of tablets.
34. The kit of 33, wherein at least one of the tablets comprises about 100 mg to about 300 mg of the *S*-[2-([[1-(2-ethylbutyl)cyclohexyl]carbonyl]amino)phenyl] 2-methylpropanethioate.
35. The kit of 33 or 34, wherein at least one of the tablets comprises about 1 mg to about 80 mg of an HMG CoA reductase inhibitor.
36. The kit of 35, wherein at least one of the tablets comprises about 5 mg to about 80 mg of an HMG CoA reductase inhibitor.
37. A method for the treatment or prophylaxis of a cardiovascular disorder in a patient, which comprises treating the patient with a therapeutically effective amount of a combination of (a) *S*-[2-([[1-(2-ethylbutyl)cyclohexyl]carbonyl]amino)phenyl] 2-methylpropanethioate or a prodrug that forms *S-*[2-([[1-(2-ethylbutyl)cyclohexyl]carbonyl]amino)phenyl] thiol *in vivo,* and (b) at least one HMG CoA reductase inhibitor.

## Claims

1. A combination comprising (a) *S*-[2-([[1-(2-ethylbutyl)cyclohexyl]carbonyl]amino) phenyl] 2-methylpropanethioate, and (b) at least one HMG CoA reductase inhibitor.

2. The combination of claim 1, wherein the HMG CoA reductase inhibitor is selected from the group consisting of atorvastatin, pravastatin, fluvastatin, simvastatin, lovastatin, rosuvastatin, pitavastatin, and pharmaceutically acceptable salts and hydrates thereof.

3. The combination of claim 2, wherein the HMG CoA reductase inhibitor is atorvastatin calcium, pravastatin sodium, fluvastatin sodium, simvastatin, lovastatin, or rosuvastatin calcium.

4. A pharmaceutical composition comprising (a) *S*-[2-([[1-(2-ethylbutyl) cyclohexyl]carbonyl]amino)phenyl] 2-methylpropanethioate, (b) at least one HMG CoA reductase inhibitor, and (c) one or more pharmaceutically acceptable carriers.

5. The pharmaceutical composition of claim 4, wherein the HMG CoA redutase inhibitor is selected from the group consisting of atorvastatin, pravastatin, fluvastatin, simvastatin, lovastatin, rosuvastatin, and pharmaceutically acceptable salts and hydrates thereof.

6. The pharmaceutical composition of any of claims 4 or 5, wherein the *S*-[2-([[1-(2-ethylbutyl)cyclohexyl]carbonyl]amino)phenyl] 2-methylpropanethioate is present in an amount of 100 mg to 300 mg.

7. A package comprising separate dosage units, of which (a) at least one dosage unit comprises *S*-[2-([[1-(2-ethylbutyl)cyclohexyl]carbonyl]amino)phenyl] 2-methylpropanethioate, and (b) at least one other dosage unit comprises an HMG CoA reductase inhibitor.

8. The combination or pharmaceutical composition of claims 1 to 6 for use in the treatment or prophylaxis of a cardiovascular disorder in a patient.

9. The combination or pharmaceutical composition for use according to claim 8, wherein the HMG CoA reductase inhibitor is to be administered to the patient in an amount of 1 mg to 80 mg per day.

10. The combination or pharmaceutical composition for use according to claim 8 or 9, wherein *S*-[2-([[1-(2-ethylbutyl)cyclohexyl]carbonyl]amino)phenyl] 2-methylpropanethioate is to be administered to the patient in an amount of 300 mg to 900 mg per day.

11. The combination or pharmaceutical composition for use according to claims 8 to 10, wherein the cardiovascular disorder is selected from the group consisting of cardiovascular disease, coronary heart disease, coronary artery disease, hypertriglyceridemia, and hypercholesterolemia.

12. The combination or pharmaceutical composition for use according to any of claims 8 to 10, wherein the cardiovascular disorder is atherosclerosis.

13. The combination or pharmaceutical composition for use according to any of claims 8 to 10, wherein the cardiovascular disorder is hypoalphalipoproteinemia, hyperbetalipoproteinemia, hyperlipidemia, primary hypercholesterolemia and/or mixed dyslipidemia.

14. The combination or pharmaceutical composition for use according to any of claims 8 to 10, wherein the HDL-C level of the patient is about 60 mg/dL or less prior to initiating the treatment or prophylaxis.

## Patentansprüche

1. Kombination, umfassend
- (a) *S*-[2-([[1-(2-Ethylbutyl)cyclohexyl]carbonyl]amino)phenyl]-2-methylpropanthioat und (b) mindestens einen HMG-CoA-Reduktasehemmer.

2. Kombination gemäß Anspruch 1, wobei der HMG-CoA-Reduktasehemmer ausgewählt ist aus der Gruppe bestehend aus Atorvastatin, Pravastatin, Fluvastatin, Simvastatin, Lovastatin, Rosuvastatin, Pitavastatin und pharmazeutisch verträglichen Salzen und Hydraten davon.

3. Kombination gemäß Anspruch 2, wobei der HMG-CoA-Reduktasehemmer Atorvastatin-Calcium, Pravastatin-Natrium, Fluvastatin-Natrium, Simvastatin, Lovastatin oder Rosuvastatin-Calcium ist.

4. Arzneimittel, umfassend (a) *S*-[2-([[1-(2-Ethylbutyl)cyclohexyl]carbonyl]-amino)phenyl]-2-methylpropanthioat, (b) mindestens einen HMG-CoA-Reduktasehemmer und (c) einen oder mehrere pharmazeutisch verträgliche(n) Träger.

5. Arzneimittel gemäß Anspruch 4, wobei der HMG-CoA-Reduktasehemmer ausgewählt ist aus der Gruppe bestehend aus Atorvastatin, Pravastatin, Fluvastatin, Simvastatin, Lovastatin, Rosuvastatin und pharmazeutisch verträglichen Salzen und Hydraten davon.

6. Arzneimittel gemäß einem der Ansprüche 4 oder 5, wobei *S*-[2-([[1-(2-Ethylbutyl)cyclohexyl]carbonyl]amino)phenyl]-2-methylpropanthioat in einer Menge von 100 mg bis 300 mg vorliegt.

7. Packung, umfassend getrennte Dosiseinheiten, von denen (a) mindestens eine Dosiseinheit *S*-[2-([[1-(2-Ethylbutyl)cyclohexyl]carbonyl]amino)phenyl]-2-methylpropanthioat umfasst und (b) mindestens eine andere Dosiseinheit einen HMG-CoA-Reduktasehemmer umfasst.

8. Kombination oder Arzneimittel gemäß den Ansprüchen 1 bis 6 zur Verwendung bei der Behandlung oder Prophylaxe einer kardiovaskulären Erkrankung bei einem Patienten.

9. Kombination oder Arzneimittel zur Verwendung gemäß Anspruch 8, wobei der HMG-CoA-Reduktasehemmer dem Patienten in einer Menge von 1 mg bis 80 mg pro Tag zu verabreichen ist.

10. Kombination oder Arzneimittel zur Verwendung gemäß Anspruch 8 oder 9, wobei *S*-[2-([[1-(2-Ethylbutyl)cyclohexyl]carbonyl]amino)phenyl]-2-methylpropanthioat dem Patienten in einer Menge von 300 mg bis 900 mg pro Tag zu verabreichen ist.

11. Kombination oder Arzneimittel zur Verwendung gemäß den Ansprüchen 8 bis 10, wobei die kardiovaskuläre Erkrankung ausgewählt ist aus der Gruppe bestehend aus Herz-Kreislauf-Erkrankung, koronarer Herzerkrankung, Koronararterienerkrankung, Hypertriglyceridämie und Hypercholesterinämie.

12. Kombination oder Arzneimittel zur Verwendung gemäß einem der Ansprüche 8 bis 10, wobei die kardiovaskuläre Erkrankung Atherosklerose ist.

13. Kombination oder Arzneimittel zur Verwendung nach einem der Ansprüche 8 bis 10, wobei die kardiovaskuläre Erkrankung Hypoalphalipoproteinämie, Hyperbetalipoproteinämie, Hyperlipidämie, primäre Hypercholesterinämie und/oder gemischte Dyslipidämie ist.

14. Kombination oder Arzneimittel zur Verwendung nach einem der Ansprüche 8 bis 10, wobei der HDL-C-Spiegel des Patienten vor Beginn der Behandlung oder der Prophylaxe ungefähr 60 mg/dl oder niedriger ist.

## Revendications

1. Combinaison comprenant (a) un 2-méthylpropanethioate de *S*-[2-([[1-(2-éthylbutyl)cyclohexyl]carbonyl]amino)phényle], et (b) au moins un inhibiteur d'HMG CoA réductase.

2. Combinaison selon la revendication 1, dans laquelle l'inhibiteur d'HMG CoA réductase est choisi dans le groupe constitué par l'atorvastatine, la pravastatine, la fluvastatine, la simvastatine, la lovastatine, la rosuvastatine, la pitavastatine, et leurs sels et hydrates acceptables sur le plan pharmaceutique.

3. Combinaison selon la revendication 2, dans laquelle l'inhibiteur d'HMG CoA réductase est l'atorvastatine calcique, la pravastatine sodique, la fluvastatine sodique, la simvastatine, la lovastatine, ou la rosuvastatine calcique.

4. Composition pharmaceutique comprenant (a) un 2-méthylpropanethioate de *S-*[2-([[1-(2-éthylbutyl)cyclohexyl]carbonyl]amino)phényle], (b) au moins un inhibiteur d'HMG CoA réductase, et (c) un ou plusieurs véhicules acceptables sur le plan pharmaceutique.

5. Composition pharmaceutique selon la revendication 4, dans laquelle l'inhibiteur d'HMG CoA réductase est choisi dans le groupe constitué par l'atorvastatine, la pravastatine, la fluvastatine, la simvastatine, la lovastatine, la rosuvastatine, et leurs sels et hydrates acceptables sur le plan pharmaceutique.

6. Composition pharmaceutique selon l'une quelconque des revendications 4 ou 5, dans laquelle le 2-méthylpropanethioate de *S*-[2-([[1-(2-éthylbutyl)cyclohexyl]carbonyl]amino)phényle] est présent en une quantité de 100 à 300 mg.

7. Emballage comprenant des doses unitaires distinctes, dans lequel (a) au moins une dose unitaire comprend le 2-méthylpropanethioate de *S*-[2-([[1-(2-éthylbutyl)cyclohexyl]carbonyl]amino)phényle], et (b) au moins une autre dose unitaire comprend un inhibiteur d'HMG CoA réductase.

8. Combinaison ou composition pharmaceutique selon les revendications 1 à 6 pour une utilisation dans le traitement ou la prévention d'un trouble cardiovasculaire chez un patient.

9. Combinaison ou composition pharmaceutique pour une utilisation selon la revendication 8, dans laquelle l'inhibiteur d'HMG CoA réductase doit être administré au patient en une quantité de 1 à 80 mg par jour.

10. Combinaison ou composition pharmaceutique pour une utilisation selon la revendication 8 ou 9, dans laquelle le 2-méthylpropanethioate de *S*-[2-([[1-(2-éthylbutyl)cyclohexyl]carbonyl]amino)phényle] doit être administré au patient en une quantité de 300 à 900 mg par jour.

11. Combinaison ou composition pharmaceutique pour une utilisation selon les revendications 8 à 10, dans laquelle le trouble cardiovasculaire est sélectionné dans le groupe constitué de maladie cardiovasculaire, coronaropathie, insuffisance coronaire, hypertriglycéridémie, et hypercholestérolémie.

12. Combinaison ou composition pharmaceutique pour une utilisation selon l'une quelconque des revendications 8 à 10, dans laquelle le trouble cardiovasculaire est l'athérosclérose.

13. Combinaison ou composition pharmaceutique pour une utilisation selon l'une quelconque des revendications 8 à 10, dans laquelle le trouble cardiovasculaire est l'hypoalphalipoprotéinémie, l'hyperbêtalipoprotéinémie, l'hyperlipidémie, l'hypercholestérolémie primaire et/ou la dyslipidémie mixte.

14. Combinaison ou composition pharmaceutique pour une utilisation selon l'une quelconque des revendications 8 à 10, dans laquelle le niveau des HDL-C du patient est d'environ 60 mg/dL ou moins avant l'initiation du traitement ou de la prophylaxie.
